Europäisches Patentamt

European Patent Office

Office européen des brevets

(19)

(11) Publication number: **0 319 315**
**A2**

# EUROPEAN PATENT APPLICATION

(12)

(21) Application number: **88311437.3**

(22) Date of filing: **02.12.88**

(51) Int. Cl.4: **C 07 K 7/00**
**A 61 K 37/02**

(30) Priority: **04.12.87 US 128815**

(43) Date of publication of application:
**07.06.89 Bulletin 89/23**

(84) Designated Contracting States:
**AT BE CH DE ES FR GB GR IT LI LU NL SE**

(71) Applicant: **SCRIPPS CLINIC AND RESEARCH
FOUNDATION
10666 North Torrey Pines Road
La Jolla California 92037-1093 (US)**

(72) Inventor: **Zimmerman, Theodore S.
544 Bonair Street
La Jolla California 92037 (US)**

**Houghten, Richard A.
558 Ford Avenue
Solona Beach California 92075 (US)**

**Foster, Paul A.
8507 Capricorn Way Apt. No. 7
San Diego California 92126 (US)**

**Fulcher, Carol A.
7418 Faye Avenue
La Jolla California 92037 (US)**

(74) Representative: **Jump, Timothy John Simon et al
VENNER, SHIPLEY & CO. 368 City Road
London EC1V 2QA (GB)**

(54) **The von Willebrand factor binding domain of factor VIII.**

(57) Peptides that inhibit the binding of von Willebrand Factor to Factor VIII which can be used for preventing thrombosis and purifying Factor VIII. Methods for preparing these peptides and purifying Factor VIII using these peptides are also disclosed. Also disclosed are mutants of human Factor VIII molecules which can be used for alleviating Factor VIII deficiency resulting from von Willebrand Factor inhibitory activity.

EP 0 319 315 A2

**Description**

## THE VON WILLEBRAND FACTOR BINDING DOMAIN OF FACTOR VIII

This invention relates to peptides which inhibit the binding of Factor VIII (FVIII) to von Willebrand factor (vWF).

The invention also relates to the production of recombinant DNA produced mutant FVIII molecules with reduced or absent capacity to bind to vWF.

vWF and FVIII both have important but different functions in the maintenance of hemostasis. vWF participates in platelet-vessel wall interactions at the site of vascular injury whereas FVIII accelerates the activation of Factor X by Factor IXa in the presence of platelets and calcium ions. vWF and FVIII circulate in plasma as a noncovalently linked complex thought to be held together by both electrostatic and hydrophobic forces. It has been demonstrated that vWF stabilizes FVIII in plasma in both in vitro and in vivo systems and prolongs its half-life in the circulation. Consequently, in the absence of endogeneous vWF the circulating half-life of FVIII is markedly reduced. Since FVIII participates in the intrinsic pathway of blood coagulation, agents capable of interfering with the interaction of FVIII and vWF would alter the FVIII level in plasma and in this manner serve as anti-thrombotic agents. The peptides of the present invention have the ability to act as anti-thrombotic agents by their prevention of the binding of vWF to FVIII. Also the recombinant DNA produced mutant FVIII molecules of the present invention have the ability to provide therapy to patients with circulating inhibitory antibodies to vWF.

Various methods have been developed for the purification of FVIII. However, there is a need for improved FVIII purification methods which result in higher yields of FVIII. The present invention accomplishes this by using peptides based on the amino acid sequence of FVIII containing the vWF binding domain for the purification of FVIII. This allows FVIII to be purified using milder conditions, resulting in an improved yield of FVIII.

The present invention provides a peptide or Factor VIII polypeptide fragment which inhibits binding of von Willebrand Factor to Factor VIII and reacts with a monoclonal anti-Factor VIII antibody C4 and a polyclonal anti-Factor VIII peptide antibody 2645 capable of specifically binding to the region of Factor VIII containing the von Willebrand Factor binding domain which contains the following amino acid sequence:
LQSDQEEIDYDDTISVEMKKEDFDIYDEDENQSPRSFQKK.

The invention further provides a peptide or Factor VIII polypeptide fragment which inhibits binding of von Willebrand Factor to Factor VIII and reacts with a monoclonal anti-Factor VIII antibody C4 and a polyclonal anti-Factor VIII peptide antibody 2645 capable of specifically binding to the region of Factor VIII containing the von Willebrand Factor binding domain which contains a sequential subset of the following amino acid sequence:
LQSDQEEIDYDDTISVEMKKEDFDIYDEDENQSPRSFQKK.

The invention may comprise a peptide or Factor VIII polypeptide fragment which inhibits binding of von Willebrand Factor to Factor VIII and reacts with a monoclonal anti-factor VIII antibody C4 and a polyclonal anti-Factor VIII peptide antibody 2645 capable of specifically binding to the region of Factor VIII containing the von Willebrand Factor binding domain which contains the following amino acid sequence:
VEMKKEDFDIYDEDE.

The invention may further comprise a peptide or Factor VIII polypeptide fragment which inhibits binding of von Willebrand Factor to Factor VIII and reacts with a monoclonal anti-Factor VIII antibody C4 and a polyclonal anti-Factor VIII peptide antibody 2645 capable of specifically binding to the region of Factor VIII containing the von Willebrand Factor binding domain which contains a sequential subset of the following amino acid sequence:
VEMKKEDFDIYDEDE.

The invention may comprise a peptide having the following amino acid sequence:
LQSDQEEIDYDDTISVEMKKEDFDIYDEDENQSPRSFQKK
which inhibits binding of von Willebrand Factor to Factor VIII, whose amino acid sequence is that of amino acid residues 1655-1694 of the Factor VIII sequence and reacts with a monoclonal anti-Factor VIII antibody C4 and a polyclonal anti-Factor VIII peptide antibody 2645 capable of specifically binding to the region of Factor VIII containing the von Willebrand Factor binding domain.

The invention may comprise a peptide comprising a sequential subset of a peptide having the following amino acid sequence:
LQSDQEEIDYDDTISVEMKKEDFDIYDEDENQSPRSFQKK
which inhibits binding of von Willebrand Factor to Factor VIII, whose amino acid sequence is that of amino acid residues 1655-1694 of the Factor VIII sequence and reacts with a monoclonal anti-Factor VIII antibody C4 and a polyclonal anti-Factor VIII peptide antibody 2645 capable of specifically binding to the region of Factor VIII containing the von Willebrand Factor binding domain.

The invention may comprise a peptide having the following amino acid sequence:
VEMKKEDFDIYDEDE
which inhibits binding of von Willebrand Factor to Factor VIII, whose amino acid sequence is that of amino acid residues 1670-1684 of the Factor VIII sequence and reacts with a monoclonal anti-Factor VIII antibody C4 and a polyclonal anti-Factor VIII peptide antibody 2645 capable of specifically binding to the region of Factor VIII containing the von Willebrand Factor binding domain.

The invention may comprise a peptide comprising a sequential subset of a peptide having the following amino acid sequence:
VEMKKEDFDIYDEDE
which inhibits binding of von Willebrand Factor to Factor VIII, whose amino acid sequence is that of amino acid residues 1670-1684 of the Factor VIII sequence and reacts with a monoclonal anti-Factor VIII antibody C4 and a polyclonal anti-Factor VIII peptide antibody 2645 capable of specifically binding to the region of Factor VIII containing the von Willebrand Factor binding domain.

The invention may further comprise a mutant of human Factor VIII with deletions of the von Willebrand Factor binding site or alterations in the von Willebrand Factor Binding site or deletions or alterations in the Factor VIII sequence which effect the von Willebrand Factor binding site resulting in decreased or absent von Willebrand Factor binding.

The invention may further comprise a mutant of human Factor VIII with deletions of the von Willebrand Factor binding site or alterations in the von Willebrand Factor binding site within amino acid residues 1655-1694 or deletions or alterations in the Factor VIII sequence which effect the von Willebrand Factor binding site resulting in decreased or absent von Willebrand Factor binding.

The invention may further comprise a mutant of human Factor VIII with deletions of the von Willebrand Factor binding site or alterations in the von Willebrand Factor binding site within amino acid residues 1670-1684 or deletions or alterations in the Factor VIII sequence which effect the von Willebrand Factor binding site resulting in decreased or absent von Willebrand Factor binding.

The invention may further comprise therapeutic compositions containing amounts of peptides effective to inhibit binding of von Willebrand Factor to Factor VIII in a patient, in association with a pharmaceutically acceptable carrier.

The invention may further comprise a method of inhibiting thrombosis in a patient by administering to the patient an effective amount of these peptides.

The invention may further comprise therapeutic compositions containing mutants of human Factor VIII effective to treat Factor VIII deficiency in a patient resulting from von Willebrand Factor inhibitory activity, in association with a pharmaceutically acceptable carrier.

The invention may further comprise a method of alleviating Factor VIII deficiency resulting from von Willebrand Factor inhibitory activity in a patient by administering to the patient an effective amount of these mutant Factor VIII molecules.

The invention may further comprise improved methods for the preparation of purified Factor VIII using peptides based on the amino acid sequence of Factor VIII containing the von Willebrand Factor binding domain and any sequential subset of these peptides.

As indicated the present invention encompasses polypeptide fragments and synthetic peptides that inhibit the binding of FVIII to vWF, whose amino acid sequences are that of fragments of FVIII and react with a monoclonal anti-FVIII antibody C4 and a polyclonal anti-Factor VIII peptide antibody 2645 capable of specifically binding to the region of FVIII containing the vWF binding domain.

In addition, the present invention identifies the FVIII sequences VEMKKEDFDIYDEDE (1670-1684)

and LQSDEEIDYDDTISVEMKKEDFDIYDEDENQSPRSFQKK (1655-1694) as binding domains of FVIII for vWF. These findings provide the basis for the design of FVIII synthetic peptides with the ability to competitively displace FVIII from vWF. Such peptides are of potential use in displacing FVIII from vWF at physiological ionic strength. In vitro this displacement can be used to purify FVIII or vWF free from the other molecule. Such displacement in vivo can be used as an anti-thrombotic intervention by reducing the half-life of circulating FVIII.

Development of FVIII peptides capable of dissociating the FVIII-vWF complex is useful in the improvement of techniques for the purification of FVIII from plasma and commercial FVIII concentrates. Some methods depend on the elution of FVIII from vWF bound to anti-vWF antibodies coupled to a support medium with buffers containing high salt concentrations such as 0.3M $CaCl_2$ which interfere in the coagulation assay of the product and also result in an added desalting step. Elution with VEMKKEDFDIYDEDE and LQSDEEIDYDDTISVEMKKEDFDIYDEDENQSPRSFQKK or subsets of them could eliminate this problem. In addition, dissociation of FVIII from vWF in plasma with these peptides would allow purification directly from plasma by a FVIII monoclonal antibody. This would allow recovery of the approximately 40-50% of FVIII which is lost in the cryoprecipitation step. Antibodies to VEMKKEDFDIYDEDE or a subset of it can be used to separate unactivated FVIII from thrombin activated FVIII. VEMKKEDFDIYDEDE resides on a portion of the FVIII molecule which is cleaved from the light chain by thrombin. Thus only uncleaved light chain would bind to antibodies to VEMKKEDFDIYDEDE then during immunoaffinity chromatography. VEMKKEDFDIYDEDE or a subset of it could be used for elution.

Dissociation of FVIII from vWF with VEMKKEDF-DIYDEDE or LQSDEEIDYDDTISVEMKKEDFDIYDE-DENQSPRSFQKK or monoclonal or polyclonal anti-FVIII antibodies such as C4 and 2645 can be used as an anti-thrombotic therapy by causing a decrease in FVIII levels and the induction of an anticoagulant state.

Synthetic recombinant DNA produced FVIII with deletions of the vWF binding site or alterations in the vWF binding site resulting in decreased or absent vWF binding may be useful in the treatment of patients with acquired inhibitory antibodies to vWF with little or no measurable vWF or FVIII activity. Infusion of FVIII devoid of vWF binding capability would not be affected by such an anti-vWF antibody and therapeutic FVIII concentrations can be achieved to partially correct the patients' bleeding diathesis.

The monoclonal anti-FVIII antibody C4 was found to have blocked the binding of FVIII to vWF in radioactive labelling and ELISA systems. The epitope of C4 must reside close to that of the vWF binding domain of FVIII. The C4 antibody was therefore used as a market of the vWF binding domain.

Competitive inhibition assay of FVIII binding to solid-phase vWF was performed with 5 ug of whole unreduced vWF in 1 ml of 0.01 M PO$_4$, 0.15 M NaCl, 0.02% NaN$_3$, pH 7.3 (PBS), incubated with three 1/4 inch in diameter polystyrene beads (Pierce Chemical Company, Rockford, IL) per 16mm in diameter tissue culture well for 2 hours at room temperature. The solution was removed and the wells and the beads were blocked with 1 ml of PBS containing 0.05% Tween-20 and 3% human serum albumin for 1 hour at room temperature. The wells and the beads were then stored in this solution at 4°C until use. The wells and beads were washed x 2 prior to use with PBS 0.05% Tween-20 and incubated for 1 1/2 hours at room temperature with 1.5 units of purified FVIII and 0-200 ug of monoclonal anti-FVIII antibody C4 or 0-400 ug of polyclonal anti-FVIII antibody 2645 in 1 ml of 0.05M imidazole, 0.15 M NaCl, 0.02% NaN$_3$, pH 7.0, 3 mM CaCl$_2$. The beads were then washed x 3 with PBS 0.05% Tween-20 and incubated 1 1/2 hours at room temperature with 1.2-2.4 x 10$^6$ cpm of $^{125}$I-monoclonal anti-FVIII antibody C2 (specific activity 8.3-9.9 x 10$^9$ cpm/mg) or 2.4-4.9 x 10$^6$ cpm of $^{125}$I-monoclonal anti-FVIII antibody C5 (specific activity 1.2-3.2 x 10$^{10}$ cpm/mg) in 1 ml of PBS 0.05% Tween-20 containing 0.5% bovine gamma globulin. After incubation the wells and beads were washed with PBS 0.05% Tween-20 x 2. The beads were transferred to clean wells and washed an additional two times and separately counted for bound radioactivity. Radioactivity was measured by a gamma counter.

The failure of $^{125}$I-C2 or C5 to bind indicated that C4 or 2645 had blocked the binding of FVIII to vWF. Both C4 and 2645 inhibited the binding of FVIII to vWF in a dose dependant manner. In a typical experiment, total counts bound of $^{125}$I-C2, indicating binding to FVIII complexed to immobilized vWF, was 4226 cpm (above background counts). When C4 in a concentration of 10 ug/ml was added to the FVIII solution and then incubated with the polystyrene beads, there was a reduction in counts bound to 1947 cpm or a 54% reduction in the amount of FVIII bound to vWF. This effect of C4 was shown to not involve inhibition of $^{125}$I-C2 binding since a similar reduction in counts bound was seen when $^{125}$I-C5 was used. C5 binds to a site on the heavy chain of FVIII and not on the light chain where C2 binds and where the binding domain of vWF resides. When the concentration of C4 was increased to 100 ug/ml, there was near total inhibition of FVIII binding with total counts measured of 27 cpm above background.

The following table summarizes the inhibition of FVIII binding resulting from the addition of increased concentrations of C4:

| C4 (ug/ml) | % inhibition | CPM |
|---|---|---|
| 0 | 0% | 4226 |
| 10 | 54% | 1947 |
| 20 | 63% | 1577 |
| 50 | 91% | 485 |
| 100 | 100% | 27 |
| 200 | 100% | 0 |

When the polyclonal anti-FVIII peptide antibody 2645 was added to the FVIII incubation step at a concentration of 50 ug/ml there was a reduction in bound counts from 4146 to 2698 cpm. When a concentration of 300 ug/ml was added, the counts bound were reduced to 300 cpm above background. The following table summarizes the inhibition of FVIII binding resulting from the addition of increased concentrations of polyclonal antibody 2645:

| 2645 (ug/ml) | % inhibition | CPM |
|---|---|---|
| 0 | 0% (by definition) | 4146 |
| 20 | 20% | 3326 |
| 50 | 35% | 2698 |
| 100 | 59% | 1706 |
| 200 | 84% | 669 |
| 300 | 93% | 300 |
| 400 | 99% | 25 |

The addition of monoclonal antibody made against the FVIII synthetic peptide composed of amino acid residues 1660-1674 up to a concentration of 200 ug/ml or a polyclonal antibody made against a FVIII synthetic peptide composed of amino acid residues 1680-1694 up to a concentration of 400 ug/ml produced no significant inhibition of FVIII binding. Thus the vWF binding domain of FVIII lies within VEMKKEDFDIYDEDE (1670-1684) or a subset of it.

The localization of the binding epitope of C4 was demonstrated to also be contained within VEMKKEDFDIYDEDE (1670-1684) by the competitive inhibition of the binding of C4 to FVIII by this peptide in an ELISA system. In this system, purified human FVIII was diluted to approximately 3 units per ml in 20 mM Tris-HCl, 0.02% NaN$_3$, pH 9.6 and 100 ul added to each 1.0 x 0.6 cm well of a multi-well tissue culture plate (Linbro, Flow Laboratories Inc.) and incubated at room temperature for 2 hours. The plates were then stored in this solution at 4°C until use. Prior to use, the solution was removed and the wells were blocked with 200 ul of 20 mM Tris-HCl, 0.02% NaN$_3$, 1% bovine serum albumin (BSA) for 1 hour at room temperature. After blocking, the solution was removed and the wells were washed x 3 with 250 ul of a buffer containing 0.14 M NaCl, 0.0015 M KH$_2$PO$_4$, 0.0084 M Na$_2$HPO$_4$, 0.05% Nonidet P-40 (washing buffer).

Alternatively, purified FVIII was diluted in 0.01 M NaHCO$_3$ and 100 ul added to each well. The plates were then desiccated at room temperature and the plates stored at 4°C until use. Prior to use the wells were blocked and washed as described above. 100 ul of sample solution containing monoclonal antibody C4 and FVIII peptide was then added to each well. C4 at final concentration of 0.1 to 1 ug/ml and FVIII peptide at a final concentration of 1nM to 1mM in washing buffer with 1% BSA were preincubated for 2 hours at 37°C or overnight at 4°C prior to addition to the wells. After a 1 hour incubation at room temperature the sample solution was removed and the wells washed x 3 with 250 ul of washing buffer. 100 ul of peroxidase goat anti-mouse IgG (H + L) Zymed Laboratories, Inc.) diluted 1:2000 with washing buffer with 1% BSA was added to each well and incubated for 1 hour at room temperature. The solution was then removed and the wells washed x 4 with 250 ul of washing buffer. 100 ul of 0-Phenylenediamine substrate (Zymed) dissolved 0.024 M citric acid, 0.051 M $Na_2HPO_4$, 0.03% hydrogen peroxide was added to each well and incubated at room temperature for 5 to 10 minutes before being quenched with 100 ul of 2M $H_2SO_4$. The color change in each well was read on a MR 600 Microplate Reader (Dynatech Instruments, Inc.).

In this system, the absence of color indicated the blocking of antibody binding by VEMKKEDFDIYDEDE (1670-1684). In a typical experiment, C4 in a concentration of 0.7 ug/ml bound to FVIII and produced an absorbance reading (OD) of 0.869. The pre-incubation of C4 with VEMKKEDFDIYDEDE (1670-1684) at a concentration of 1uM resulted in a decrease in the measured OD to 0.044 representing a 95% reduction in antibody binding to FVIII. Furthermore, peptides consisting of amino acid residues 1665-1679 and 1680-1694 were unable to significantly block the binding of C4 to FVIII. At a concentration of 1 uM these peptides reduced C4 binding by only 15% and 21% respectively.

Peptides based on the amino acid sequence of Factor VIII and a sequential subset of at least three amino acid residues of the peptide are synthesized as described by Houghten et al., Proc. Natl. Acad. Sci. 82:5135 (1985).

In the well known procedure for solid-phase synthesis of a peptide, the desired peptide is assembled starting from an insoluble support such as benzhydryl amine or chloromethylated resin (derived from cross-linked polystyrene, and available from chemical supply houses). The amino acid at the carboxy-terminal end of the desired polypeptide, carrying protecting groups on the alpha-amino nitrogen and on any other reactive sites, is attached to the resin from solution using known peptide coupling techniques. The protecting group on the alpha-amino group is removed (leaving other protecting groups, if any, intact), and the next amino acid of the desired sequence (carrying suitable protecting groups) is attached, and so on. When the desired polypeptide has been completely built up, it is cleaved from the resin support, all protecting groups are removed, and the polypeptide is recovered. Examples of suitable protecting groups are: alpha-tert-butyloxycarbonyl for the alpha-amino-group; benzyl, 4-methoxybenzyl, or 4-methylbenzyl for the thiol group of cysteine, the beta-carboxylic acid group of aspartic acid, the gamma-carboxylic acid group of glutamic acid and the hydroxyl group of serine, threonine, and tyrosine; benzyloxycarbonyl or a 2-chloro- or 3, 4-dimethoxy-derivative thereof for the ring nitrogens of histidine and tryptophan and the epsilon-amino group of lysine; p-nitrophenyl for the amide nitrogens of asparagine and glutamine; and nitro or tosyl for the guanidine group of arginine.

For purposes of this disclosure, accepted shorthand designations of the amino acids have been used. A complete listing is provided herein below:

One and three-letter Amino Acid Abbreviations

| A | Ala | Alanine |
|---|---|---|
| C | Cys | Cysteine |
| D | Asp | Aspartic Acid |
| E | Glu | Glutamic Acid |
| F | Phe | Phenylalanine |
| G | Gly | Glycine |
| H | His | Histidine |
| I | Ile | Isoleucine |
| K | Lys | Lysine |
| L | Leu | Leucine |
| M | Met | Methionine |
| N | Asn | Asparagine |
| P | Pro | Proline |
| Q | Gln | Glutamine |
| R | Arg | Arginine |
| S | Ser | Serine |
| T | Thr | Threonine |
| V | Val | Valine |
| W | Trp | Tryptophan |
| Y | Tyr | Tyrosine |
| B | Asx | Asp or Asn, not distinguished |
| Z | Glx | Glu or Gln, not distinguished |
| X | X | Undetermined or atypical amino acid |

Mutant Factor VIII molecules can be produced by recombinant DNA techniques with deletions of the vWF binding site or alterations in the vWF binding site resulting in decreased or absent vWF binding. First, the linear amino acid sequence defining the antibody binding site for vWF is determined. This is accomplished by determining the smallest proteolytic fragment of Factor VIII to which the antibody binds. Once the sequence is known, synthetic peptides based upon the amino acid sequence of these fragments are synthesized and individual amino acids are systematically deleted or substituted. From the reactivity of the antibodies with these sequences, the critical amino acids are determined. Once the critical region of the epitope is known, mutant Factor VIII molecules can be produced in which the substitution or deletion is made in the critical region of the epitope.

To make the desired Factor VIII mutant polypeptide by recombinant DNA techniques, the gene for human Factor VIII is cloned, treated by various methods involving site-directed mutagenesis, inserted into a cell, and used to express the Factor VIII. For a description of the gene and how to use it, see Gitschier, J. et al., "Characterization of the Human Factor VIII Gene," Nature, Vol. 312, pp. 326-330; Wood, William I, et al., "Expression of Active Human Factor VIII from Recombinant DNA Clones," Nature, Vol. 312, pp. 330-337; and Toole, John J., et al., "Molecular Cloning of a cDNA Encoding Human Antihemophilic Factor", Nature, Vol. 312, pp. 342-347. Various methods of site directed mutagenesis may be used, for instance, single base pair changes at cytosine residues can be created using chemicals such as bisulfite, which deaminates cytosine to uracil, which base pairs like thymine. For a description of directed mutagenesis with sodium bisulfite, see Shortle, David, et al., "Directed Mutagenesis", Ann. Rev. Genetics, Vol. 15, pp. 265-294. Alternatively, oligonucleotide-directed mutagenesis may be used. For a description, see Zoller, Mark J. and Smith, Michael, "Oligonucleotide Directed Mutagenesis of DNA Fragments Cloned into M13 Vectors," Methods In Enzymology, Recombinant DNA, Part B, Vol. 100, pp. 468-500.

The polyclonal antibody 2645 had been raised by immunization of a rabbit with the peptide VEMKKEDFDIYDEDE (1670-1684). The synthetic VEMKKEDFDIYDEDE peptide containing amino acid residues 1670-1684 from the FVIII sequence was synthesized as described above. The synthetic peptide was then coupled to a carrier protein, keyhole limpet hemocyanin (KLH), as follows:

1. Weigh out the desired amount of peptide and an equal amount of carrier;

2. Dissolve peptide and carrier protein in phosphate buffered saline (PBS) and dilute to a final concentration of 2 mg of carrier per ml;

3. Add 124 ul of working glutaraldehyde per 1 ml of carrier-peptide solution;

4. Stir overnight at room temperature;

5. Dialyze at least 6 hours against distilled water;

6. Lyophilize;

7. Weigh recovered material and determine the percent coupled.

90% recovery of carrier protein was assumed. For example, starting with 20 mg of carrier and 20 mg of peptide, 30 mg was recovered after dialyzing and lyophilizing. Of that 30 mg, 18 mg was carrier protein and 12 mg was peptide.

One milligram of coupled peptide in complete Freund's adjuvant was injected subcutaneously into a New Zealand white rabbit in multiple injection sites. Three booster immunizations of one mg coupled peptide in incomplete Freund's adjuvant were given at weekly intervals in the same manner. The animal was bled two weeks after the last injection and every two weeks thereafter.

Purification of the rabbit antibody was by affinity chromatography on protein A - Sepharose. See, Ey, et al., "Isolation of Pure IgG1, IgG2a, and IgG2b Immunoglobulins from Mouse Serum Using Protein A Sepharose", Immunochemistry 15: 429-436 (1978). A 3 ml sample of rabbit antiserum was diluted 1:5 in 0.14 molar sodium phosphate buffer (pH 8) and applied to 9.5 cm x 1 cm column of protein A-Sepharose (Pharmacia). The column was then washed with this buffer to remove nonabsorbed proteins. The rabbit IgG was eluted from the column with 0.10 molar soduim citrate buffer (pH3). The protein peak was dialyzed against 0.05 molar tris [HCl], 0.15 molar sodium chloride, 0.02% sodium azide (pH 7.35).

The monoclonal antibody C4 (previously described in Fulcher et al., Human Factor VIII Procoagulant Protein: Monoclonal Antibodies Define Precursor-Product Relationships and Functional Epitopes, J. Clin. Invest., Vol. 76, pp. 117-124 (1985)) is an antibody for FVIII:C which inhibits the binding of Factor VIII to vWF.

To produce the hybridoma cell line producing monoclonal antibody C4, Balb/c mice were immunized by intraperitoneal injection of 1 ug of purified Factor VIII:C in complete Freund's adjuvant. 10- and 50-ug boosters in incomplete Freund's adjuvant were given at 1-wk intervals. The final 100-ug booster was given without adjuvant 3 days before fusion. At 4 wk, the mouse spleen cells were fused with P3X63 mouse plasmacytoma cells as described in Brown et al., J. Biol. Chem. 255:4980-4983 (1980). Cell culture and production of monoclonal antibody in mouse ascites fluid were essentially by Liu et al., J. Immunol. 124:2728-2736 (1980). The antibodies were selected using a solid-phase assay in Linbro-Titertek (Flow Laboratories, Inglewood, CA) plates and an enzyme-linked immunosorbent detection system as described in Engvall and Perlmann, Immunochemistry 8:871-874 (1971) using a peroxidase-antibody conjugate (Zymed Laboratories, Burlingame, CA). The plates were coated with 100 ng of purified Factor VIII:C. Clones were also screened against plates coated with 100 ng of human fibrinogen, plates coated with 100 ng of human fibronectin, and plates coated with 100 ng of human von Willebrand factor per well, each protein being a potential contaminant of the immunogen.

Clones which were positive only on the Factor VIII:C-coated plates were also tested for their ability to inhibit plasma Factor VIII:C activity, using a modified Kasper assay (see Kasper et al., Thromb. Diath. Haemorrh. 34:869-872 (1975)). Culture supernatants were incubated with an equal volume of normal pooled human plasma, which had been diluted 1:10 or 1:20 into assay buffer as described in Fulcher et al., Proc. Natl. Acad. Sci. 79:1648-1652 (1982) to which had been added 2% (vol/vol) of 0.5 M sodium citrate, pH 6.5. Incubation was for 2 h at 37°C, after which samples were assayed for Factor VIII:C procoagulant activity. Culture supernatants from clone C4 inhibited 75% of the residual plasma Factor VIII:C activity, as compared with control (non-anti-Factor VIII:C supernatant) incubations.

Culture supernatants were tested for immunoglobulin class and subclass by double diffusion in agarose gels using commercially available antisera (Miles Laboratories, Inc., Elkhart, IN). Protein A-Sepharose chromatography (Pharmacia Fine Chemicals, Piscataway, NJ) of mouse ascites fluid was as described by Ey et al., Immunochemistry 15:479-436 (1978). Purified antibodies were concentrated in a pressure ultrafiltration stirred cell (Amicon Corp., Danvers, MA) using a YM-10 membrane, aliquoted, and stored frozen at -70°C. Protein A-Sepharose purified monoclonal antibodies were assayed for their ability to inhibit plasma Factor VIII:C procoagulant activity using the modified Kasper assay (see Kasper et al., Thromb. Diath. Haemorrh. 34:869-872 (1975)).

One or more of the peptides of the present invention can be formulated into pharmaceutical preparations for therapeutic, diagnostic, or other uses. To prepare them for intraveneous administration, the compositions are dissolved in water containing physiologically compatible substances such as sodium chloride (e.g. 0.35 - 2.0 M), glycine, and the like and having a buffered pH compatible with physiological conditions. The amount to administer for the prevention of thrombosis will depend on the severity with which the patient is subject to thrombosis, but can be determined readily for any particular patient.

## Claims

1. A peptide or Factor VIII polypeptide fragment which inhibits binding of von Willebrand Factor to Factor VIII and reacts with a monoclonal anti-Factor VIII antibody C4 and a polyclonal anti-Factor VIII peptide antibody 2645 capable of specifically binding to the region of Factor VIII containing the von Willebrand Factor binding domain which contains the following amino acid sequence:
LQSDQEEIDYDDTISVEMKKEDFDIYDEDENQSPRSFQKK,
or a sequential subset thereof.

2. A peptide or Factor VIII polypeptide fragment which inhibits binding of von Willebrand Factor to Factor VIII and reacts with a monoclonal anti-Factor VIII antibody C4 and a polyclonal anti-Factor VIII peptide antibody 2645 capable of specifically binding to the region of Factor VIII containing the von Willebrand Factor binding domain which contains the following amino acid sequence:
VEMKKEDFDIYDEDE,
or a sequential subset thereof.

3. A peptide having the following amino acid sequence:
LQSDQEEIDYDDTISVEMKKEDFDIYDEDENQSPRSFQKK,
or a sequential subset thereof which inhibits binding of von Willebrand Factor to Factor VIII, whose amino acid sequence is that of amino acid residues 1655-1694 of the Factor VIII sequence and reacts with a monoclonal anti-Factor VIII antibody C4 and a polyclonal anti-Factor VIII peptide antibody 2645 capable of specifically binding to the region of Factor VIII containing the von Willebrand Factor binding domain.

4. A peptide having the following amino acid sequence:
VEMKKEDFDIYDEDE,
or a sequential subset thereof, which inhibits binding of von Willebrand Factor to Factor VIII, whose amino acid sequence is that of amino acid residues 1670-1684 of the Factor VIII sequence and reacts with a monoclonal anti-Factor VIII antibody C4 and a polyclonal anti-Factor VIII peptide antibody 2645 capable of specifically binding to the region of Factor VIII containing the von Willebrand Factor binding domain.

5. A mutant of human Factor VIII with deletions of the von Willebrand Factor binding site or alterations in the von Willebrand Factor binding side or or deletions or alterations in the Factor VIII sequence which effect the von Willebrand Factor binding site resulting in decreased or absent von Willebrand Factor binding.

6. A mutant of human Factor VIII with deletions of the von Willebrand Factor binding site or alterations in the von Willebrand Factor binding site within amino acid residues 1655-1694 or deletions or alterations in the Factor VIII sequence which effect the von Willebrand Factor binding site resulting in decreased or absent von Willebrand Factor binding.

7. A mutant of human Factor VIII with delections of the von Willebrand Factor binding site or alterations in the von Willebrand Factor binding site within amino acid residues 1670-1684 or deletions or alterations in the Factor VIII sequences which effect the von Willebrand Factor binding site resulting in decreased or absent von Willebrand Factor binding.

8. A therapeutic composition comprising an amount of a peptide or Factor VIII polypeptide fragment according to any of claims 1-4 effective to inhibit binding of von Willebrand Factor to factor VIII in a patient, in association with a pharmaceutically acceptable carrier.

9. A method of inhibiting thrombosis in a patient suffering therefrom, comprising administering to such patient an effective amount of a product according to any of claims 1-4.

10. A therapeutic composition comprising an amount of a mutant of human Factor VIII according to any of claims 5-7 effective to treat Factor VIII deficiency in a patient resulting from von Willebrand Factor inhibitory activity, in association with a pharmaceutically acceptable carrier.

11. A method of alleviating Factor VIII deficiency resulting from von Willebrand Factor inhibitory activity in a patient suffering therefrom, comprising administering to such patient an effective amount of a product according to any of claims 5-7.

12. An improved method of preparing Factor VIII comprising:
(a) absorbing Factor VIII from a recombinant produced, plasma or commercial concentrate source onto particles bound to a monoclonal antibody raised against a peptide based on the amino acid sequence of Factor VIII containing the von Willebrand Factor binding domain and any sequential subset of said peptide;
(b) eluting the absorbed Factor VIII; and
(c) recovering the highly purified Factor VIII.

13. The method according to claim 12 wherein said monoclonal antibody is raised against a peptide of the following amino acid sequence; LQSDQEEIDYDDTISVEMKKEDFDIYDEDENQSPRSFQKK which amino acid sequence is that of amino acid residues 1655-1694 of the Factor VIII sequence; a peptide of the following amino acid sequence:
VEMKKEDFDIYDEDE
whose amino acid sequence is that of amino acid residues 1670-1684 of the Factor VIII sequence; or any sequential subset of either of said peptides.

14. An improved method of preparing Factor VIII comprising:

(a) absorbing Factor VIII from a recombinant-produced, plasma or commercial concentrate source onto particles bound to a monoclonal antibody which binds Factor VIII;

(b) eluting the absorbed Factor VIII with a peptide based on the amino acid sequence of Factor VIII containing the von Willebrand Factor binding domain and any sequential subset of said peptide; and

(c) recovering the highly purified Factor VIII.

15. The method according to claims 12 or 14 wherein the eluant used in step (b) is a peptide of the following amino acid sequence:
LQSDQEEIDYDDTISVEMKKEDFDIYDEDENQSPRSFQKK
whose amino acid sequence is that of amino acid residues 1655-1694 of the Factor VIII sequence; a peptide of the following amino acid sequence:
VEMKKEDFDIYDEDE
whose amino acid sequence is that of amino acid residues 1670-1684 of the Factor VIII sequence; or any sequential subset of either of said peptides.

16. The method according to claim 14 wherein the eluant used in step (b) is a peptide based on the amino acid sequence of Factor VIII containing the von Willebrand Factor binding domain and any sequential subset of said peptide.

17. A method according to claim 14 wherein the eluant used in step (b) is a saline solution, and preferably calcium chloride.

18. The method according to claims 34 or 37 wherein said absorbent particles in step (a) are agarose.